# EUROPEAN PATENT SPECIFICATION

(11) **EP 3 683 580 B1**
(45) Date of publication and mention of the grant of the patent: **18.12.2024**
(21) Application number: 18912008.2
(22) Date of filing: 12.07.2018
(51) Int. Cl.: G01N 33/76, G01N 21/78, G01N 21/84, G01N 21/77, G01N 33/68

(54) **ELECTRONIC DETECTION PEN AND EARLY PREGNANCY WEEK NUMBER DETECTION METHOD**
ELEKTRONISCHER NACHWEISSTIFT UND VERFAHREN ZUM NACHWEISDER FRÜHSCHWANGERSCHAFTSWOCHE
STYLO DE DÉTECTION ÉLECTRONIQUE ET MÉTHODE DE DÉTECTION PRÉCOCE DU NOMBRE DE SEMAINES DE GROSSESSE

(30) Priority: 26.03.2018 CN 201810253639
(43) Date of publication of application: 22.07.2020
(73) Proprietor: Nantong Egens Biotechnology Co., Ltd., Nantong, Jiangsu 226010 (CN)
(72) Inventor: OU, Weijun, Nantong, Jiangsu 226010 (CN); SUN, Yipin, Nantong, Jiangsu 226010 (CN); XU, Yan, Nantong, Jiangsu 226010 (CN)
(74) Representative: Lavoix
(86) International application number: PCT/CN2018/095411
(87) International publication number: WO 2019/184144

(56) References cited:
- WO-A1-2010/055355
- WO-A1-2016/156981
- CN-A- 106 932 598
- CN-A- 106 932 598
- CN-U- 203 479 811
- CN-U- 204 330 782
- CN-U- 205 749 530
- CN-U- 206 573 593
- CN-U- 206 573 593
- CN-U- 206 876 717
- CN-U- 207 081 741
- GB-A- 2 460 660
- US-A1- 2008 241 958
- US-A1- 2009 305 436
- US-A1- 2010 129 935
- US-A1- 2015 010 441
- DATABASE WPI Week 201777, 20 October 2017 Derwent World Patents Index; AN 2017-72850F, XP002801003

## Description

### CROSS REFERENCE TO RELATED APPLICATIONS

This application claims priority to Chinese Patent Application No. 201810253639.9 filed on March 26, 2018, entitled "electronic detection pen and early pregnancy week number detection method".

### TECHNICAL FIELD

The present application relates to a field of human chorionic gonadotropin detection, in particular to an electronic detection pen and early pregnancy week number detection method.

### BACKGROUND

Human chorionic gonadotropin (hCG) is a glycoprotein hormone secreted by placental trophoblast cells during pregnancy. It consists of two different subunits, α and β connected by a non-covalent bond. During production, secretion, and metabolism of hormones, hCG molecules will undergo various changes such as rupture and dissociation, so as to exist in various molecular forms in blood and urine. hCG is the only placenta hormone that does not increase with the increase of placental weight. A concentration of hCG is 5-50 mmIU/ml in the blood and >25 mIU/ml in the urine after 1 week of pregnancy, reaches a peak around 8-10 weeks, and lasts for 1-2 weeks then decreases rapidly, and then gradually decreases and maintains at about 1/5-1/10 of the peak level until delivery. Therefore, early pregnancy can be detected by detecting the hCG concentration in blood and urine.

At present, a test paper in a general early pregnancy test pen is mostly used by a double-antibody sandwich method, which uses colloidal gold as an indicator to detect the hCG concentration in urine, such method can only be used to determine whether pregnancy or not, not pregnancy days. The method for detecting the pregnancy days is mainly blood test, ultrasonic detection, and combination of HCG detection and ultrasonic detection, which require professionals to obtain a detection result after a long period of testing, and have a complicated operation. WO 2016/156981 A1 discloses a test device to detect pregnancy in a human female subject, the test device comprising: an assay means to measure the absolute or relative amount of hCG m a sample from the subject; an assay means to measure the absolute or relative amount of FSH in a sample from the subject; and m assay means to measure the absolute or relative amount of one or mere progesterone metabolites I» a sample from the subject.

GB 2460660 A discloses a method for determining a quantitative estimate of the length of time since conception in a female mammalian subject, the method comprising: a) providing a liquid sample suspected of containing human chorionic gonadotrophin (hCG); b) measuring, by means of an assay or assay device, an analyte measurement signal, whose value is dependent upon the level of hCG;c) comparing the measured signal value to an analyte threshold, wherein said analyte threshold corresponds to a time since conception; providing a quantitative estimate of the length of time since conception based upon the comparison in step (c), and also discloses an assay device, an assay result display device and a dataset for use in the above. The device comprises a common porous sample receiver comprising two assay flow paths, optical means for reading the assay and power source and associated electronics, the flow paths have respective control zones, and detection zones the two paths may have low and high sensitivities respectively.

### SUMMARY

Therefore, the technical problem to be solved by the present application is to solve the problem that an early pregnancy electronic detection pen in the prior art can only detect whether pregnancy or not, and cannot detect pregnancy times.

In order to solve the above problem, according to a first aspect, the present application provides an electronic detection pen according to claim 1. The electronic detection pen according to the invention comprises a housing, a test paper, and a control circuit, wherein the test paper is used for measuring a concentration of a human chorionic gonadotropin in urine, and the test paper comprises a first test paper and a second test paper, wherein a minimum detectable quantity of the human chorionic gonadotropin of the first test paper is less than that of the human chorionic gonadotropin of the second test paper; and the control circuit is used for obtaining a detection result of an early pregnancy week number according to a chromogenic reaction of the first test paper and a chromogenic reaction of the second test paper, and outputting the detection result by means of a liquid crystal display (LCD-4) connected to the control circuit; wherein the first test paper and the second test paper each include: a substrate, and a sample pad, a colloidal gold adsorption pad, an antibody carrying film and a water absorption pad sequentially adhered to the substrate;wherein the loading pad, the colloidal gold adsorption pad, the antibody carrying membrane, and the water absorption pad are only in contact with and only partially overlap with adjacent pad thereof; the antibody carrying film has a quality control line and a detection line arranged at an interval, the quality control line is close to the water absorption pad, and the detection line is close to the colloidal gold adsorption pad;the colloidal gold adsorption pad of the first test paper has adsorbed colloidal gold-anti-human chorionic gonadotropin β-hCG monoclonal antibody conjugate, and the colloidal gold adsorption pad of the second test paper has absorbed colloidal gold-anti-human chorionic gonadotropin β-hCG monoclonal antibody conjugates and a free β antibody;the quality control line is coated with an anti-mouse IgG polyclonal antibody; the detection line is coated with an anti-human chorionic gonadotropin a-hCG monoclonal antibody; the control circuit comprises:a light emitting diode for providing a light source and irradiating the quality control line and the detection line of the test paper; a photoelectric sensor for receiving reflected lights from the quality control line and the detection line of the test paper and generating an electrical signal having an intensity proportional to that of the reflected lights; a control chip for receiving the electric signal and calculating an early pregnancy week number according to the intensity of the electric signal; and a power source for supplying power to the light emitting diode, the photoelectric sensor, and the control chip; the electronic detection pen being characterized in that the photoelectric sensor comprises: a first photoelectric sensor and a second photoelectric sensor for receiving reflected lights from the quality control line and the detection line of the first test paper respectively; and a third photoelectric sensor and a fourth photoelectric sensor for receiving reflected lights from the quality control line and the detection line of the second test paper respectively.

Optionally, the minimum detectable quantity of the human chorionic gonadotropin of the first test paper is not higher than 25 mIU/ml, and the minimum detectable quantity of the human chorionic gonadotropin of the second test paper is not higher than 100 mIU/ml.

Optionally, the sample pad and the water absorption pad are each provided with a protective film.

Optionally, the test paper is disposed in the housing, and the sample pad of the test paper extends outside the housing for absorbing urine of a person to be tested.

Moreover, according to the second aspect, the present application provides a method for detecting an early pregnancy week number, according to claim 5. The method for detecting an early pregnancy week number, is characterized by comprising steps of: (S100) taking urine of a person to be tested, and soaking the test paper of the electronic detection pen according to the invention into the urine; (S200) generating different chromogenic reactions for the first test paper and the second test paper under an action of human chorionic gonadotropin in the urine; (S300) obtaining a detection result of an early pregnancy week number according to the chromogenic reaction and sending the detection result to LCD by a control circuit; and (S400) outputting the detection result to the LCD.

Optionally, in the method the control circuit obtains the detection result of an early pregnancy week number according to the chromogenic reaction, and sends the detection result to the LCD, which includes the steps of: providing a light source and irradiating the quality control line and the detection line of the test paper by the light emitting diode; receiving reflected lights from the test paper, generating an electrical signal with an intensity corresponding to the intensity of the reflected lights and sending the electric signal to the control chip by the photoelectric sensor; and obtaining the detection result of an early pregnancy week number according to the intensity of the electrical signal, and sending the detection result to the LCD by the control chip. Optionally, when the control chip receives the electrical signals sent by the first and third photoelectric sensors and does not receive the electrical signals sent by the second and fourth photoelectric sensors, the detection result shows that the tested person is not pregnant;
when the control chip receives the electrical signals sent by the first, second and third photoelectric sensors and does not receive the electrical signal sent by the fourth photoelectric sensor, the detection result shows that the tested person is pregnant and has a pregnancy time of 1-2 weeks;
when the control chip receives the electrical signals sent by the first, second, third and fourth photoelectric sensors, and an intensity of the electrical signal sent by the second photoelectric sensor is far greater than that of the fourth photoelectric sensor, the detection result shows that the tested person is pregnant and has a pregnancy time of 2-3 weeks;
when the control chip receives the electrical signals sent by the first, second, third and fourth photoelectric sensors, and an intensity of the electrical signal sent by the second photoelectric sensor is similar to that of the fourth photoelectric sensor, the detection result shows that the tested person is pregnant and has a pregnancy time of more than 3 weeks;
when the control chip does not receive the electrical signals sent by the first, second, third and fourth photoelectric sensors, the detection result shows that the test paper is invalid.

The technical solutions provided by the present application have the following advantages.
1. A electronic detection pen provided in the present application comprises a housing, a test paper, and a control circuit. The test paper is used for measuring a concentration of a human chorionic gonadotropin in urine, and the test paper comprises a first test paper and a second test paper, wherein a minimum detectable quantity of the human chorionic gonadotropin of the first test paper is less than that of the human chorionic gonadotropin of the second test paper; and the control circuit is used for obtaining a detection result of an early pregnancy week number according to a chromogenic reaction of the first test paper and a chromogenic reaction of the second test paper, and outputting the detection result by means of a liquid crystal display (LCD) connected to the control circuit. By providing the first test paper and the second test paper with different minimum detectable quantities of the human chorionic gonadotropin, when testing a person's urine with a certain concentration of hCG, the first test paper and the second test paper produce different chromogenic reactions, thus the hCG concentration in human urine tested can be deduced by means of the reaction comparison of the first test paper and the second test paper, and the hCG concentration in the urine during the early pregnancy period of a pregnant woman is associated with the pregnancy time, so that the early pregnancy week number of the pregnant woman can be detected.
2. In the electronic detection pen provided in the present application, a photoelectric sensor comprises: a first photoelectric sensor and a second photoelectric sensor for receiving reflected lights from the quality control line and the detection line of the first test paper respectively; and a third photoelectric sensor and a fourth photoelectric sensor for receiving reflected lights from the quality control line and the detection line of the second test paper respectively. By providing the first, second, third and fourth photoelectric sensors, an electrical signal is generated by receiving reflected lights from the detection lines of the first test paper and the second test paper, so as to obtain an early pregnancy week number detection result, and an electrical signal is generated by receiving reflected lights from the quality control lines of the first test paper and the second test paper, so as to obtain the detection result on whether the first test paper and the second test paper is invalid or not, thus reducing the possibility of detection result has error due to the failure of the test papers, thereby improving the detection accuracy of the electronic detection pen.
3. A method for detecting an early pregnancy week number provided in the present application comprises steps of: taking urine of a person to be tested, and soaking the test paper of the electronic detection pen according to the invention. into the urine; generating different chromogenic reactions for the first test paper and the second test paper under an action of human chorionic gonadotropin in the urine; obtaining a detection result of an early pregnancy week number according to the chromogenic reaction and sending the detection result to LCD by a control circuit; and outputting the detection result to the LCD. The method for detecting an early pregnancy week number can detect the early pregnancy week number of the tested person, solving problems that the method for detecting an early pregnancy in the prior art can only detect whether the tested person is pregnant or not, but a professional is still needed to determine the pregnancy time by a long-term test, thereby reducing the detection steps and reducing the detection cost.

### DESCRIPTION OF THE DRAWING

In order to more clearly illustrate the technical solutions of the embodiments of the present application or the prior art, the drawings used in the embodiments of the present application or the prior art will be briefly described below. Obviously, the drawings in the following description are only some embodiments of the present application, and those skilled in the art can obtain other drawings based on these drawings without any creative efforts.
Fig. 1 is a schematic view showing a structure of an electronic detection pen provided in Embodiment 1 of the present application;
Fig. 2 is a schematic view showing a circuit structure of a control circuit of an electronic detection pen provided in Embodiment 1 of the present application;
Fig. 3 is a flowchart of a method for detecting an early pregnancy week number provided in Embodiment 2 of the present application;
Fig. 4 is a schematic view of performing step S 100 of a method for detecting an early pregnancy week number detection provided in Embodiment 2 of the present application;
Fig. 5 is a schematic flowchart of step S300 of a method for detecting an early pregnancy week number detection provided in Embodiment 2 of the present application;

In the figures, the reference numerals are as follows:
1-housing; 11-pen cap; 12-urine detection line; 2-control circuit; 21-light emitting diode; 22-photoelectric sensor; 23- control chip; 24-power supply; 3-test paper; 4- liquid crystal display (LCD).

### DETAILED DESCRIPTION OF THE EMBODIMENTS

The technical solutions of the present application will be described clearly and completely with reference to the accompanying drawings. It is obvious that the described embodiments are only a part of the embodiments of the present application, but not all of the embodiments. All other embodiments obtained by those skilled in the art based on the embodiments of the present application without any creative efforts are within the scope of the present application.

In the description of the present application, the terms "first" and "second" and the like are used for descriptive purposes only and are not to be construed as indicating or implying relative importance.

### Embodiment 1

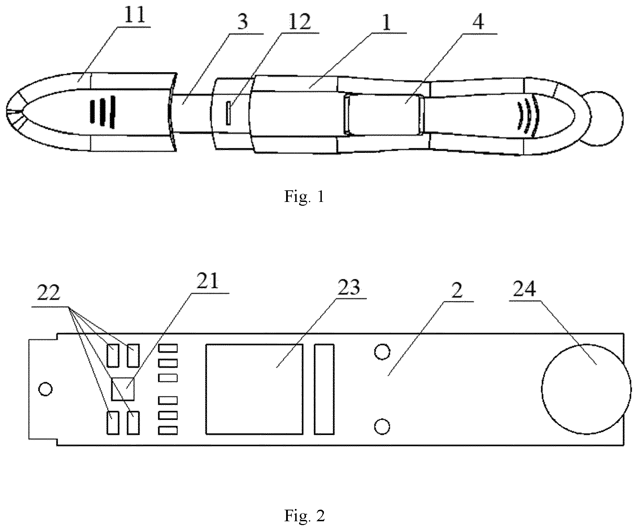

Embodiment 1 provides an electronic detection pen, as shown in Fig. 1, the electronic detection pen comprises a housing 1, a control circuit 2 and a test paper 3. The test paper 3 is used for measuring a concentration of a human chorionic gonadotropin in urine. The test paper 3 comprises a first test paper and a second test paper, wherein a minimum detectable quantity of the human chorionic gonadotropin of the first test paper is less than that of the human chorionic gonadotropin of the second test paper. The control circuit 2 is used for obtaining a detection result of an early pregnancy week number according to a chromogenic reaction of the first test paper and a chromogenic reaction of the second test paper, and the detection result is output by means of a liquid crystal display (LCD) 4 connected to the control circuit 2. In the Embodiment 1, the minimum detectable quantity of the human chorionic gonadotropin of the first test paper is not higher than 25 mIU/ml, and the minimum detectable quantity of the human chorionic gonadotropin of the second test paper is not higher than 100 mIU/ml. The above minimum detectable quantity of the human chorionic gonadotropin of the first and second test papers is established by referring to the reference value of the urine hCG concentration of pregnant women during different periods of early pregnancy described in the *"Chinese Clinical Test Operating Procedures",* and can be adjusted according to actual needs of application or according to manufacturing experience in a specific embodiment. In this embodiment, the LCD 4 is disposed on an outer wall of the housing 1 or inside a LCD window opened on the housing 1.

In this embodiment, both control circuit 2 and test paper 3 are provided inside the housing 1. A sample pad of the test paper 3 extends outside the housing 1 to absorb the urine of the tested person. In a specific embodiment, the housing 1 is provided with a urine detection line 12 on one end near the sample pad of the test paper 3. When the test paper 3 is immersed in the urine of the tested person, the electronic detection pen should be immersed in a position of the urine detection line 12 which is specifically arranged according to the length of the sample pad.

In Embodiment 1, as shown in Fig. 2, the control circuit 2 comprises a light emitting diode 21, a photoelectric sensor 22, a control chip 23 and a power supply 24. The light emitting diode 21 is used for providing a light source and irradiating the quality control line and the detection line of the test paper 3. The photoelectric sensor 22 is used for receiving reflected lights from the quality control line and the detection line of the test paper 3 and generating an electrical signal having an intensity proportional to that of the reflected lights. The control chip 23 is used for receiving the electric signal and calculating an early pregnancy week number according to the intensity of the electric signal. The power source 24 is used for supplying power to the light emitting diode 21, the photoelectric sensor 22, and the control chip 23. In this embodiment, the photoelectric sensor 22 comprises: a first photoelectric sensor and a second photoelectric sensor for receiving reflected lights from the quality control line and the detection line of the first test paper respectively; and a third photoelectric sensor and a fourth photoelectric sensor for receiving reflected lights from the quality control line and the detection line of the second test paper respectively. By providing the first, second, third and fourth photoelectric sensors, an electrical signal is generated by receiving reflected lights from the detection lines of the first test paper and the second test paper, so as to obtain an early pregnancy week number detection result, and an electrical signal is generated by receiving reflected lights from the quality control lines of the first test paper and the second test paper, so as to obtain the detection result on whether the first test paper and the second test paper is failed or not, thus reducing the possibility of detection result error due to the failure of the test papers, and improving the detection accuracy of the electronic detection pen.

In this embodiment, the first test paper and the second test paper each include: a substrate, and a sample pad, a colloidal gold adsorption pad, an antibody carrying film and a water absorption pad sequentially adhered to the substrate; wherein the loading pad, the colloidal gold adsorption pad, the antibody carrying membrane, and the water absorption pad are only in contact with and only partially overlap with adjacent pad thereof; the antibody carrying film has a quality control line and a detection line arranged at an interval, the quality control line is close to the water absorption pad, and the detection line is close to the colloidal gold adsorption pad. The sample pad and the water absorption pad are each provided with a protective film. The colloidal gold adsorption pad of the first test paper has adsorbed colloidal gold-anti-human chorionic gonadotropin β-hCG monoclonal antibody conjugate, and the colloidal gold adsorption pad of the second test paper has absorbed colloidal gold-anti-human chorionic gonadotropin β-hCG monoclonal antibody conjugates and a free β antibody; and the quality control line is coated with an anti-mouse IgG polyclonal antibody; the detection line is coated with an anti-human chorionic gonadotropin α-hCG monoclonal antibody.

In the electronic detection pen provided in this embodiment, by providing the first test paper and the second test paper with different minimum detectable quantities of the human chorionic gonadotropin, when testing the person's urine with a certain concentration of hCG, the first test paper and the second test paper produce different chromogenic reactions, thus the hCG concentration in human urine tested can be deduced by means of the reaction comparison of the first test paper and the second test paper, and the hCG concentration in the urine during the early pregnancy period of a pregnant woman is associated with the pregnancy time, so that the early pregnancy week number of the pregnant woman can be detected.

### Embodiment 2

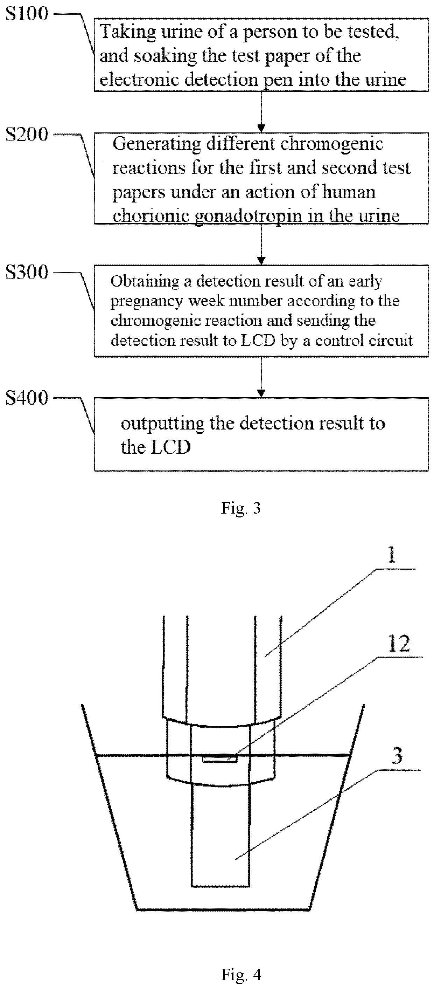

Embodiment 2 provides a method for detecting an early pregnancy week number, as shown in Fig. 3, the method comprises the following steps.

Step S100: urine of a person to be tested is took, and the test paper of the electronic detection pen of Embodiment 1 is soaked into the urine. In this embodiment, as shown in Fig. 4, firstly, the urine sample is collected by a clean and dry container, then a pen cap 11 of the electronic detection pen is removed, the sample pad extending outside the housing 1 is immersed into the urine, until a position of the urine detection line 12 on the housing 1 is immersed, so that the sample pad absorbs enough urine samples. In a specific embodiment, the immersion time of the sample pad is preferably 15-25 seconds. In this embodiment, the sample pad extending outside the housing 1 can also be placed in the urine stream for more than 5 seconds to realize an absorption of the urine sample. It should be noted that when using this method to absorb the urine sample, other parts of the electronic pen should not get wet, so as not to influence the normal operation of the control circuit 2 in the electronic detection pen.

Step S200: different chromogenic reactions of the first test paper and the second test paper are generated under an action of human chorionic gonadotropin in the urine. In this embodiment, when a complex, formed by the human chorionic gonadotropin in the urine sample combining with the colloidal gold-anti-human chorionic gonadotropin β-hCG monoclonal antibody conjugate adsorbed on the first test paper, passes through the detection line of the first test paper, it binds to a pre-coated α-hCG antibody, thus obtaining a chromogenic reaction showing a visible prunosus band is accumulated on the detection line by a principle of double antibody sandwich method and immunochromatography. In this embodiment, when the human chorionic gonadotropin in the urine sample, and the colloidal gold-anti-human chorionic gonadotropin β-hCG monoclonal antibody conjugate and free β antibody adsorbed on the second test paper, transfer to a chromatographic membrane at the same time, and then bind to a pre-coated α-hCG antibody as passing through the detection line of the second test paper, thus obtinaing a chromogenic reaction showing a visible prunosus band is accumulated on the detection line by a principle of double antibody sandwich method and immunochromatography. In this embodiment, since the minimum detectable quantity of the human chorionic gonadotropin of the first test paper is different from that of the second test paper, the chromogenic reactions of the first test paper and the second test paper are different when detecting the same urine sample.

Step S300: a detection result of an early pregnancy week number is obtained according to the chromogenic reaction, and the detection result is sent to LCD by a control circuit.

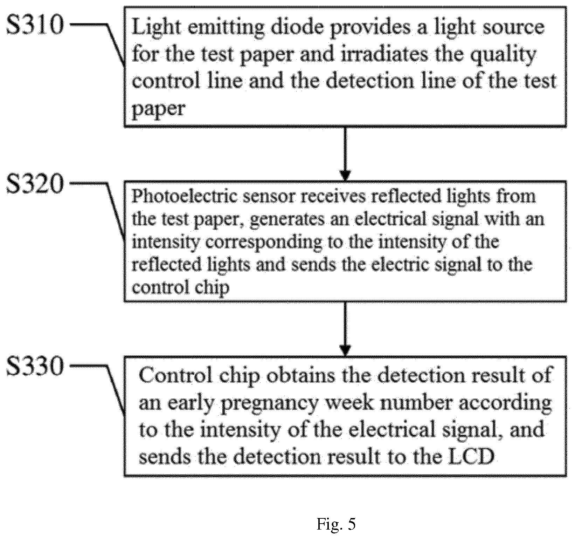

In Embodiment 2, the control circuit 2 comprises a light emitting diode 21, a photoelectric sensor 22, a control chip 23, and a power source 24. As shown in Fig. 5, the step S300 includes the following steps.

Step S310: the light emitting diode provides a light source for the test paper and irradiates the quality control line and the detection line of the test paper. In this embodiment, the power supply 24 is used to supply power to the light emitting diode 21, and the light emitting diode 21 is used to provide a light source for the test paper 3, so that the photoelectric sensor 22 can receive the reflected light from the test paper 3, and then step S320 will be performed.

Step S320: the photoelectric sensor receives reflected lights from the test paper, generates an electrical signal with an intensity corresponding to the intensity of the reflected lights and sends the electric signal to the control chip. In this embodiment, the electrical signal generated by the photoelectric sensor 22 has an intensity proportional to the intensity of the reflected lights from the test paper. The photoelectric sensor 22 comprises: a first photoelectric sensor and a second photoelectric sensor for receiving reflected lights from the quality control line and the detection line of the first test paper respectively; and a third photoelectric sensor and a fourth photoelectric sensor for receiving reflected lights from the quality control line and the detection line of the second test paper respectively.

Step S330: the control chip obtains the detection result of an early pregnancy week number according to the intensity of the electrical signal, and sends the detection result to the LCD.

In this embodiment, when the control chip 23 receives the electrical signals sent by the first and third photoelectric sensors and does not receive the electrical signals sent by the second and fourth photoelectric sensors, the detection result shows that the tested person is not pregnant.

When the control chip 23 receives the electrical signals sent by the first, second and third photoelectric sensors and does not receive the electrical signal sent by the fourth photoelectric sensor, the detection result shows that the tested person is pregnant and has a pregnancy time of 1-2 weeks.

When the control chip 23 receives the electrical signals sent by the first, second, third and fourth photoelectric sensors, and an intensity of the electrical signal sent by the second photoelectric sensor is far greater than that of the fourth photoelectric sensor, the detection result shows that the tested person is pregnant and has a pregnancy time of 2-3 weeks.

When the control chip 23 receives the electrical signals sent by the first, second, third and fourth photoelectric sensors, and an intensity of the electrical signal sent by the second photoelectric sensor is similar to that of the fourth photoelectric sensor, the detection result shows that the tested person is pregnant and has a pregnancy time of more than 3 weeks.

When the control chip 23 does not receive the electrical signals sent by the first, second, third and fourth photoelectric sensors, the detection result shows that the test paper is invalid.

Step S400: the detection result is outputted to the LCD.

The method for detecting an early pregnancy week number can detect the early pregnancy week number of the tested person, solving problems that the method for detecting an early pregnancy in the prior art can only detect whether the tested person is pregnant or not, but a professional is still needed to determine the pregnancy time by a long-term test, thereby reducing the detection steps and reducing the detection cost.

It is apparent that the above embodiments are merely examples for clarity of illustration, and are not intended to limit the embodiments. Other variations or modifications in various forms may be made by those skilled in the art in view of the above description. There is no need and no way to present all of the embodiments herein. The obvious variations or modifications derived therefrom are still within the scope of protection of the present application as defined by the appended claims.

## Claims

1. An electronic detection pen (11), comprising a housing (1), a test paper (3), and a control circuit (2), wherein
the test paper (3) is used for measuring a concentration of a human chorionic gonadotropin in urine, and
the test paper (3) comprises a first test paper (3) and a second test paper (3), wherein a minimum detectable quantity of the human chorionic gonadotropin of the first test paper (3) is less than that of the human chorionic gonadotropin of the second test paper (3); and
the control circuit (2) is used for obtaining a detection result of an early pregnancy week number according to a chromogenic reaction of the first test paper (3) and a chromogenic reaction of the second test paper (3), and outputting the detection result by means of a liquid crystal display (LCD-4) connected to the control circuit (2);
wherein the first test paper (3) and the second test paper (3) each include:
a substrate, and
a sample pad, a colloidal gold adsorption pad, an antibody carrying film and a water absorption pad sequentially adhered to the substrate;
wherein the loading pad, the colloidal gold adsorption pad, the antibody carrying membrane, and the water absorption pad are only in contact with and only partially overlap with adjacent pad thereof; the antibody carrying film has a quality control line and a detection line (12) arranged at an interval, the quality control line is close to the water absorption pad, and the detection line (12) is close to the colloidal gold adsorption pad;
the colloidal gold adsorption pad of the first test paper (3) has adsorbed colloidal gold-anti-human chorionic gonadotropin β-hCG monoclonal antibody conjugate, and the colloidal gold adsorption pad of the second test paper (3) has absorbed colloidal gold-anti-human chorionic gonadotropin β-hCG monoclonal antibody conjugates and a free β antibody;
the quality control line is coated with an anti-mouse IgG polyclonal antibody; the detection line (12) is coated with an anti-human chorionic gonadotropin α-hCG monoclonal antibody;
the control circuit (2) comprises:
a light emitting diode (21) for providing a light source and irradiating the quality control line and the detection line (12) of the test paper (3);
a photoelectric sensor (22) for receiving reflected lights from the quality control line and the detection line (12) of the test paper (3) and generating an electrical signal having an intensity proportional to that of the reflected lights;
a control chip (23) for receiving the electric signal and calculating an early pregnancy week number according to the intensity of the electric signal; and
a power source (24) for supplying power to the light emitting diode (21), the photoelectric sensor (22), and the control chip (23);
the electronic detection pen (11) being **characterized in that** the photoelectric sensor (22) comprises:
a first photoelectric sensor (22) and a second photoelectric sensor (22) for receiving reflected lights from the quality control line and the detection line (12) of the first test paper (3) respectively; and
a third photoelectric sensor (22) and a fourth photoelectric sensor (22) for receiving reflected lights from the quality control line and the detection line (12) of the second test paper (3) respectively.

2. The electronic detection pen (11) according to claim 1, **characterized in that**, the minimum detectable quantity of the human chorionic gonadotropin of the first test paper is not higher than 25 mIU/ml, and the minimum detectable quantity of the human chorionic gonadotropin of the second test paper (3) is not higher than 100 mIU/ml.

3. The electronic detection pen (11) according to claim 1, **characterized in that**, the sample pad and the water absorption pad are each provided with a protective film.

4. The electronic detection pen (11) according to claim 1, **characterized in that**, the test paper (3) is disposed in the housing (1), and the sample pad of the test paper extends outside the housing (1) for absorbing urine of a person to be tested.

5. A method for detecting an early pregnancy week number, **characterized by** comprising steps of:
(S100) taking urine of a person to be tested, and soaking the test paper (3) of the electronic detection pen (11) according to any one of claims 1-4 into the urine;
(S200) generating different chromogenic reactions for the first test paper (3) and the second test paper (3) under an action of human chorionic gonadotropin in the urine;
(S300) obtaining a detection result of an early pregnancy week number according to the chromogenic reaction and sending the detection result to LCD by a control circuit (2); and
(S400) outputting the detection result to the LCD.

6. The method for detecting an early pregnancy week number according to claim 5, **characterized in that**
the control circuit (2) obtains the detection result of an early pregnancy week number according to the chromogenic reaction, and sends the detection result to the LCD, which includes the steps of:
(S310) providing a light source and irradiating the quality control line and the detection line (12) of the test paper (3) by the light emitting diode (21);
(S320) receiving reflected lights from the test paper (3), generating an electrical signal with an intensity corresponding to the intensity of the reflected lights and sending the electric signal to the control chip (23) by the photoelectric sensor (22); and
(S330) obtaining the detection result of an early pregnancy week number according to the intensity of the electrical signal, and sending the detection result to the LCD by the control chip (23).

7. The method for detecting an early pregnancy week number according to claim 6, **characterized in that**:
when the control chip (23) receives the electrical signals sent by the first and third photoelectric sensors (22) and does not receive the electrical signals sent by the second and fourth photoelectric sensors (22), the detection result shows that the tested person is not pregnant;
when the control chip (23) receives the electrical signals sent by the first, second and third photoelectric sensors (22) and does not receive the electrical signal sent by the fourth photoelectric sensor (22), the detection result shows that the tested person is pregnant and has a pregnancy time of 1-2 weeks;
when the control chip (23) receives the electrical signals sent by the first, second, third and fourth photoelectric sensors (22), and an intensity of the electrical signal sent by the second photoelectric sensor (22) is far greater than that of the fourth photoelectric sensor (22), the detection result shows that the tested person is pregnant and has a pregnancy time of 2-3 weeks;
when the control chip (23) receives the electrical signals sent by the first, second, third and fourth photoelectric sensors (22), and an intensity of the electrical signal sent by the second photoelectric sensor (22) is similar to that of the fourth photoelectric sensor (22), the detection result shows that the tested person is pregnant and has a pregnancy time of more than 3 weeks;
when the control chip (23) does not receive the electrical signals sent by the first, second, third and fourth photoelectric sensors (22), the detection result shows that the test paper (3) is invalid.

## Patentansprüche

1. Elektronischer Nachweisstift (11), umfassend ein Gehäuse (1), ein Testpapier (3) und eine Steuerschaltung (2), wobei
das Testpapier (3) zum Messen einer Konzentration eines menschlichen Choriongonadotropins in Urin verwendet wird und
das Testpapier (3) ein erstes Testpapier (3) und ein zweites Testpapier (3) umfasst, wobei eine minimale nachweisbare Menge des menschlichen Choriongonadotropins des ersten Testpapiers (3) geringer ist als die des menschlichen Choriongonadotropins des zweiten Testpapiers (3); und
die Steuerschaltung (2) verwendet wird, um ein Nachweisresultat einer frühen Schwangerschaftswochenzahl gemäß einer chromogenen Reaktion des ersten Testpapiers (3) und einer chromogenen Reaktion des zweiten Testpapiers (3) zu erlangen, und um das Nachweisresultat mittels einer Flüssigkristallanzeige (LCD-4) auszugeben, die mit der Steuerschaltung (2) verbunden ist;
wobei das erste Testpapier (3) und das zweite Testpapier (3) jeweils beinhalten:
ein Substrat, und
ein Probenkissen, ein Adsorptionskissen aus kolloidalem Gold, einen antikörpertragenden Film und ein Wasserabsorptionskissen, die nacheinander auf das Substrat geklebt werden;
wobei das Beladungskissen, das Adsorptionskissen aus kolloidalem Gold, die antikörpertragende Membran und das Wasserabsorptionskissen nur in Kontakt mit einem benachbarten Kissen davon sind und sich nur teilweise überlappen; der antikörpertragende Film eine Qualitätskontrolllinie und eine Nachweislinie (12) aufweist, die in einem Abstand angeordnet sind, die Qualitätskontrolllinie nahe dem Wasserabsorptionskissen ist und die Nachweislinie (12) nahe dem Adsorptionskissen aus kolloidalem Gold ist;
das Adsorptionskissen aus kolloidalem Gold des ersten Testpapiers (3) kolloidales Gold-anti-humanes Choriongonadotropin β-hCG monoklonales Antikörperkonjugat adsorbiert hat, und das Adsorptionskissen aus kolloidalem Gold des zweiten Testpapiers (3) kolloidales Gold-anti-humanes Choriongonadotropin β-hCG monoklonale Antikörperkonjugate und einen freien β-Antikörper absorbiert hat;
die Qualitätskontrolllinie mit einem polyklonalen Anti-Maus-IgG-Antikörper beschichtet ist; die Nachweislinie (12) mit einem monoklonalen Antikörper gegen menschliches Choriongonadotropin α-hCG beschichtet ist;
wobei die Steuerschaltung (2) Folgendes umfasst:
eine Leuchtdiode (21) zum Bereitstellen einer Lichtquelle und zum Bestrahlen der Qualitätskontrolllinie und der Nachweislinie (12) des Testpapiers (3);
einen photoelektrischen Sensor (22) zum Empfangen von reflektiertem Licht von der Qualitätskontrolllinie und der Nachweislinie (12) des Testpapiers (3) und zum Erzeugen eines elektrischen Signals, das eine Intensität aufweist, die proportional zu der des reflektierten Lichts ist;
einen Steuerchip (23) zum Empfangen des elektrischen Signals und zum Berechnen einer frühen Schwangerschaftswochenzahl gemäß der Intensität des elektrischen Signals; und
eine Stromquelle (24) zum Zuführen von Energie zu der Leuchtdiode (21), dem photoelektrischen Sensor (22) und dem Steuerchips (23);
der elektronische Nachweisstift (11) **dadurch gekennzeichnet ist, dass** der photoelektrische Sensor (22) Folgendes umfasst:
einen ersten photoelektrischen Sensor (22) und einen zweiten photoelektrischen Sensor (22) zum Empfangen von reflektiertem Licht von der Qualitätskontrolllinie bzw. der Nachweislinie (12) des ersten Testpapiers (3); und
einen dritten fotoelektrischen Sensor (22) und einen vierten fotoelektrischen Sensor (22) zum Empfangen von reflektiertem Licht von der Qualitätskontrolllinie bzw. der Nachweislinie (12) des zweiten Testpapiers (3).

2. Elektronischer Nachweisstift (11) nach Anspruch 1, **dadurch gekennzeichnet, dass** die minimale nachweisbare Menge des menschlichen Choriongonadotropins des ersten Testpapiers nicht höher als 25 mIU/ml ist und die minimale nachweisbare Menge des menschlichen Choriongonadotropins des zweiten Testpapiers (3) nicht höher als 100 mIU/ml ist.

3. Elektronischer Nachweisstift (11) nach Anspruch 1, **dadurch gekennzeichnet, dass** das Probenkissen und das Wasserabsorptionskissen jeweils mit einem Schutzfilm versehen sind.

4. Elektronischer Nachweisstift (11) nach Anspruch 1, **dadurch gekennzeichnet, dass** das Testpapier (3) in dem Gehäuse (1) angeordnet ist und sich das Probenkissen des Testpapiers außerhalb des Gehäuses (1) erstreckt, um Urin einer zu testenden Person zu absorbieren.

5. Verfahren zum Nachweisen einer frühen Schwangerschaftswochenzahl, **dadurch gekennzeichnet, dass** es die folgenden Schritte umfasst:
(S100) Entnehmen von Urin einer zu testenden Person und Eintauchen des Testpapiers (3) des elektronischen Nachweisstifts (11) nach einem der Ansprüche 1-4 in den Urin;
(S200) Erzeugen unterschiedlicher chromogener Reaktionen für das erste Testpapier (3) und das zweite Testpapier (3) unter Einwirkung von humanem Choriongonadotropin in dem Urin;
(S300) Erlangen eines Nachweisresultats einer frühen Schwangerschaftswochenzahl gemäß der chromogenen Reaktion und Senden des Nachweisresultats an LCD durch eine Steuerschaltung (2); und
(S400) Ausgeben des Nachweisresultats an das LCD.

6. Verfahren zum Nachweisen einer frühen Schwangerschaftswochenzahl nach Anspruch 5, **dadurch gekennzeichnet, dass**
die Steuerschaltung (2) das Nachweisresultat einer frühen Schwangerschaftswochenzahl gemäß der chromogenen Reaktion erlangt und das Nachweisresultat an das LCD sendet, was die folgenden Schritte beinhaltet:
(S310) Bereitstellen einer Lichtquelle und Bestrahlen der Qualitätskontrolllinie und der Detektionslinie (12) des Testpapiers (3) durch die Leuchtdiode (21);
(S320) Empfangen von reflektiertem Licht von dem Testpapier (3), Erzeugen eines elektrischen Signals mit einer Intensität, die der Intensität des reflektierten Lichts entspricht, und Senden des elektrischen Signals an den Steuerchip (23) durch den photoelektrischen Sensor (22); und
(S330) Erlangen des Nachweisresultats einer frühen Schwangerschaftswochenzahl gemäß der Intensität des elektrischen Signals und Senden des Nachweisresultats durch den Steuerchip (23) an das LCD.

7. Verfahren zum Nachweisen einer frühen Schwangerschaftswochenzahl nach Anspruch 6, **dadurch gekennzeichnet, dass**
wenn der Steuerchip (23) die elektrischen Signale empfängt, die von dem ersten und dem dritten photoelektrischen Sensor (22) gesendet werden, und die elektrischen Signale, die von dem zweiten und dem vierten photoelektrischen Sensor (22) gesendet werden, nicht empfängt, das Nachweisresultat zeigt, dass die getestete Person nicht schwanger ist;
wenn der Steuerchip (23) die elektrischen Signale empfängt, die vom ersten, zweiten und dritten photoelektrischen Sensor (22) gesendet werden, und das elektrische Signal, das von dem vierten photoelektrischen Sensor (22) gesendet wird, nicht empfängt, das Nachweisresultat zeigt, dass die getestete Person schwanger ist und eine Schwangerschaftszeit von 1-2 Wochen aufweist;
wenn der Steuerchip (23) die elektrischen Signale empfängt, die vom ersten, zweiten, dritten und vierten photoelektrischen Sensor (22) gesendet werden, und eine Intensität des von dem zweiten photoelektrischen Sensor (22) gesendeten elektrischen Signals weitaus größer ist als die des vierten photoelektrischen Sensors (22), das Nachweisresultat zeigt, dass die getestete Person schwanger ist und eine Schwangerschaftszeit von 2-3 Wochen aufweist;
wenn der Steuerchip (23) die elektrischen Signale empfängt, die vom ersten, zweiten, dritten und vierten photoelektrischen Sensor (22) gesendet werden, und eine Intensität des von dem zweiten photoelektrischen Sensor (22) gesendeten elektrischen Signals ähnlich wie die des vierten photoelektrischen Sensors (22), das Nachweisresultat zeigt, dass die getestete Person schwanger ist und eine Schwangerschaftszeit von mehr als 3 Wochen aufweist;
wenn der Steuerchip (23) die elektrischen Signale, die vom ersten, zweiten, dritten und vierten photoelektrischen Sensor (22) gesendet werden, nicht empfängt, zeigt das Nachweisresultat, dass das Testpapier (3) ungültig ist.

## Revendications

1. Stylo de détection électronique (11), comprenant un boîtier (1), un papier test (3) et un circuit de commande (2), dans lequel
le papier test (3) est utilisé pour mesurer une concentration de gonadotrophine chorionique humaine dans l'urine, et
le papier test (3) comprend un premier papier test (3) et un second papier test (3), dans lequel une quantité minimale détectable de gonadotrophine chorionique humaine du premier papier test (3) est inférieure à celle de la gonadotrophine chorionique humaine du second papier test (3) ; et
le circuit de commande (2) est utilisé pour obtenir un résultat de détection d'un nombre de semaines de grossesse précoce en fonction d'une réaction chromogène du premier papier test (3) et d'une réaction chromogène du second papier test (3), et pour émettre le résultat de détection au moyen d'un écran à cristaux liquides (LCD-4) connecté au circuit de commande (2) ;
dans lequel le premier papier test (3) et le second papier test (3) comprennent chacun :
un substrat, et
un tampon d'échantillonnage, un tampon d'adsorption d'or colloïdal, un film porteur d'anticorps et un tampon d'absorption d'eau collés séquentiellement au substrat ;
dans lequel le tampon de chargement, le tampon d'adsorption d'or colloïdal, la membrane porteuse d'anticorps et le tampon d'absorption d'eau ne sont en contact qu'avec les tampons adjacents et ne les recouvrent que partiellement ; le film porteur d'anticorps comporte une ligne de contrôle de la qualité et une ligne de détection (12) disposées à un certain intervalle, la ligne de contrôle de la qualité étant proche du tampon d'absorption d'eau et la ligne de détection (12) étant proche du tampon d'adsorption d'or colloïdal ;
le tampon d'adsorption d'or colloïdal du premier papier test (3) a adsorbé le conjugué or colloïdal-anticorps monoclonal anti-gonadotrophine chorionique humaine β-hCG, et le tampon d'adsorption d'or colloïdal du second papier test (3) a absorbé les conjugués or colloïdal-anticorps monoclonal anti-gonadotrophine chorionique humaine β-hCG et un anticorps β libre ;
la ligne de contrôle de qualité est recouverte d'un anticorps polyclonal anti-IgG de souris ; la ligne de détection (12) est recouverte d'un anticorps monoclonal anti-gonadotrophine chorionique humaine α-hCG ;
le circuit de commande (2) comprenant :
une diode électroluminescente (21) pour fournir une source de lumière et irradier la ligne de contrôle de la qualité et la ligne de détection (12) du papier test (3) ;
un capteur photoélectrique (22) destiné à recevoir les lumières réfléchies par la ligne de contrôle de qualité et la ligne de détection (12) du papier test (3) et à générer un signal électrique dont l'intensité est proportionnelle à celle des lumières réfléchies ;
une puce de contrôle (23) pour recevoir le signal électrique et calculer un nombre de semaines de grossesse précoce en fonction de l'intensité du signal électrique ; et
une source d'énergie (24) pour alimenter la diode électroluminescente (21), le capteur photoélectrique (22) et la puce de contrôle (23) ;
le stylo de détection électronique (11) étant **caractérisé en ce que** le capteur photoélectrique (22) comprend :
un premier capteur photoélectrique (22) et un deuxième capteur photoélectrique (22) pour recevoir les lumières réfléchies par la ligne de contrôle de la qualité et la ligne de détection (12) du premier papier test (3) respectivement ; et
un troisième capteur photoélectrique (22) et un quatrième capteur photoélectrique (22) destinés à recevoir les lumières réfléchies par la ligne de contrôle de la qualité et la ligne de détection (12) du deuxième papier test (3), respectivement.

2. Stylo de détection électronique (11) selon la revendication 1, **caractérisé par le fait que** la quantité minimale détectable de gonadotrophine chorionique humaine du premier papier test n'est pas supérieure à 25 mUl/ml, et que la quantité minimale détectable de gonadotrophine chorionique humaine du second papier test (3) n'est pas supérieure à 100 mUl/ml.

3. Stylo de détection électronique (11) selon la revendication 1, **caractérisé par le fait que** le tampon d'échantillonnage et le tampon d'absorption d'eau sont chacun pourvus d'un film protecteur.

4. Stylo de détection électronique (11) selon la revendication 1, **caractérisé par le fait que** le papier test (3) est disposé dans le boîtier (1) et que le tampon d'échantillonnage du papier test s'étend à l'extérieur du boîtier (1) pour absorber l'urine d'une personne à tester.

5. Méthode de détection d'un nombre de semaines de grossesse précoce, **caractérisée par** les étapes suivantes :
(S100) prélèvement de l'urine d'une personne à tester et trempage dans l'urine du papier test (3) du stylo de détection électronique (11) selon l'une quelconque des revendications 1 à 4 ;
(S200) génération de réactions chromogènes différentes pour le premier papier test (3) et le second papier test (3) sous l'action de la gonadotrophine chorionique humaine dans l'urine ;
(S300) obtention d'un résultat de détection d'un nombre de semaines de grossesse précoce en fonction de la réaction chromogène et envoi du résultat de détection à l'écran LCD par un circuit de commande (2) ; et
(S400) affichage du résultat de la détection sur l'écran LCD.

6. Méthode de détection d'un nombre de semaines de grossesse précoce selon la revendication 5, **caractérisée par le fait que**
le circuit de commande (2) obtient le résultat de la détection d'un nombre de semaines de grossesse précoce en fonction de la réaction chromogène, et envoie le résultat de la détection à l'écran à cristaux liquides, ce qui comprend les étapes suivantes :
(S310) fournir une source de lumière et irradier la ligne de contrôle de qualité et la ligne de détection (12) du papier test (3) par la diode électroluminescente (21) ;
(S320) recevoir des lumières réfléchies par le papier test (3), générer un signal électrique d'une intensité correspondant à l'intensité des lumières réfléchies et envoyer le signal électrique à la puce de contrôle (23) par le capteur photoélectrique (22) ; et
(S330) obtenir le résultat de la détection d'un nombre de semaines de grossesse précoce en fonction de l'intensité du signal électrique, et envoyer le résultat de la détection à l'écran LCD par la puce de contrôle (23).

7. Méthode de détection d'un nombre de semaines de grossesse précoce selon la revendication 6, **caractérisée par le fait que**
lorsque la puce de contrôle (23) reçoit les signaux électriques envoyés par les premier et troisième capteurs photoélectriques (22) et ne reçoit pas les signaux électriques envoyés par les deuxième et quatrième capteurs photoélectriques (22), le résultat de la détection indique que la personne testée n'est pas enceinte ;
lorsque la puce de contrôle (23) reçoit les signaux électriques envoyés par les premier, deuxième et troisième capteurs photoélectriques (22) et ne reçoit pas le signal électrique envoyé par le quatrième capteur photoélectrique (22), le résultat de la détection indique que la personne testée est enceinte et que la grossesse remonte à 1 à 2 semaines ;
lorsque la puce de contrôle (23) reçoit les signaux électriques envoyés par les premier, deuxième, troisième et quatrième capteurs photoélectriques (22) et que l'intensité du signal électrique envoyé par le deuxième capteur photoélectrique (22) est nettement supérieure à celle du quatrième capteur photoélectrique (22), le résultat de la détection indique que la personne testée est enceinte et que la grossesse remonte à 2 à 3 semaines ;
lorsque la puce de contrôle (23) reçoit les signaux électriques envoyés par les premier, deuxième, troisième et quatrième capteurs photoélectriques (22) et que l'intensité du signal électrique envoyé par le deuxième capteur photoélectrique (22) est similaire à celle du quatrième capteur photoélectrique (22), le résultat de la détection indique que la personne testée est enceinte et que la grossesse remonte à plus de 3 semaines ;
lorsque la puce de contrôle (23) ne reçoit pas les signaux électriques envoyés par les premier, deuxième, troisième et quatrième capteurs photoélectriques (22), le résultat de la détection indique que le papier test (3) n'est pas valide.
